Europäisches Patentamt

(19) European Patent Office

Office européen des brevets

(11) **EP 0 663 207 B1**

(12) **EUROPEAN PATENT SPECIFICATION**

(45) Date of publication and mention
of the grant of the patent:
**11.11.1998  Bulletin 1998/46**

(51) Int. Cl.$^6$: **A61K 7/48**,  A61K 7/26

(21) Application number: **94120540.3**

(22) Date of filing: **23.12.1994**

(54) **Process for preparation of bamboo-salt, bamboo-salt**

Bamboo-Salz-Herstellungsverfahren

Procédé de préparation de sel de bambou

(84) Designated Contracting States:
**DE FR IT**

(30) Priority:  **29.12.1993 KR 9331009**
**01.09.1994 KR 9421967**

(43) Date of publication of application:
**19.07.1995  Bulletin 1995/29**

(73) Proprietor: **LUCKY LTD.**
**Seoul 150-721 (KR)**

(72) Inventors:
 • **Ha, Jae Mong**
**Chungjoo, Korea (KR)**
 • **Jeong, Kwang Lai**
**Chungjoo, Korea (KR)**
 • **Suh, Sung Soo**
**Chungjoo, Korea (KR)**

 • **Choi, Kyu Yeol**
**Chungjoo, Korea (KR)**
 • **Kim, Moon Moo**
**Chungjoo, Korea (KR)**
 • **Chang, Sug Youn**
**Chungjoo, Korea (KR)**

(74) Representative:
**Dost, Wolfgang, Dr.rer.nat., Dipl.-Chem. et al**
**Patent- und Rechtsanwälte**
**Bardehle . Pagenberg . Dost . Altenburg .**
**Frohwitter . Geissler & Partner**
**Postfach 86 06 20**
**81633 München (DE)**

(56) References cited:
**US-A- 4 915 946**          **US-A- 5 180 575**

## Description

### FIELD OF THE INVENTION

The present invention relates to a process for preparing bamboo-salt, a bamboo-salt produced by such process and a composition containing such bamboo-salt for enhancing oral hygiene. More particularly, the present invention relates to a bamboo-salt having an excellent effect of preventing and treating oral diseases and of alleviating of various inflammations and skin diseases, which is prepared by packing the bay salt into a bamboo tube, sealing the opened end of the bamboo tube with yellow earth and then calcining the bamboo tube in a furnace at high temperature, and to a process for preparation of said bamboo-salt. In addition, the present invention relates to a composition for enhancing oral hygiene, which contains said bamboo-salt as an active ingredient in combination with a conventional oral hygiene composition, and therefore, can effectively prevent and treat oral diseases, particularly periodontal diseases, and prevent teeth from decaying and further can alleviate various inflammation symptoms and skin diseases.

### BACKGROUND OF THE INVENTION

Bamboo-salt referred herein is one of the synthetic salts, which is synthesized by heating a bamboo and a salt, and has a greatly enhanced pharmacological effect through repeated treatment of the bamboo having a cytogenic function and the salt having a sterilizing and antiseptic effect in a furnace at high temperature. The bamboo-salt thus prepared has been widely used as a popular medicine for treatment of gastrointestinal disorders and various inflammation symptoms.

Numerous methods for preparing such bamboo-salt have been known and generally used. However, they are not definitely established and therefore cannot produce bamboo-salt which has a uniform composition and shows a uniform and excellent pharmacological effect.

Hereinafter, the presently known methods for preparing bamboo-salt are specifically reviewed.

According to Korean Patent Publication No. 90-1844, the salt (bay salt) is heated to 400 to 600°C in the furnace to evaporate water and to obtain a powdery salt resulting from spontaneous decomposition of the salt. The powdery salt thus obtained is mixed with a mixed leaf comprising dried and ground leaves of gingko tree, persimmon tree, pine tree and bamboo, in the weight ratio of 1:50, and the mixture is introduced into the furnace with making a fire with dried firewood such as gingko tree, persimmon tree, pine tree, bamboo, etc., wherein the leaf powders mixed with the powdery salt are burned with generating a superheat. At this time, the powdery salt is melted with the superheat generated from wood fire and burning leaves into a solution and then the melted salt solution is cooled down to crystallize the salt into which ashes of the salt and the burned firewood powder are incorporated to cause a black contaminant. The salt crystal thus obtained is ground and then repeatedly subjected 5 to 6 times to the above procedures. Finally, the obtained salt is packed into a bamboo die which is then heated to 400 to 600°C in the furnace. In this way, the bamboo die is burned to ashes and the salt is remained as a solid in the form of the bamboo die. The solidified salt thus obtained is then melted with strongly and rapidly heating to 1000 to 1300°C in the furnace. Since the melted salt contains some contaminants, it is filled in a container having great fire resistance and then completely burned with heating at 400 to 1300°C in a special electric furnace to obtain the hygienic processed salt (bamboo-salt).

The hygienic processed salt (bamboo-salt) thus prepared contains 96% of sodium chloride, 0.16% of calcium, 0.56% of magnesium, 0.88% of sulfur, 0.013% of iron, 0.005% of cupper, 37.2% of sodium, 58.8% of chlorine, etc. However, this processed salt has some disadvantages in that it is lacking in essential mineral components and does not contain each constituent in a constant amount and further the toothpaste compostion containing this bamboo-salt dose not provide a sufficient pharmacological efficacy and a satisfactory feeling of use.

Korean Early Published Patent Publication No. 90-4318 discloses a method for preparing bamboo-salt wherein the bay salt is packed into a bamboo tube; the opened end of the bamboo tube is sealed with kneaded yellow earth; the bamboo tube is calcined two times for one hour each time with charcoal fire in the furnace. The calcined salt produced during this calcining procedure is ground to powder and then the powdery salt is filled again in the bamboo tube. Then, the yellow earth is kneaded with water and the opened end, of the bamboo tube is sealed with the kneaded yellow earth. The bamboo tube is then introduced into the furnace and heated to 1000°C with a fire using pine resin as a fuel to melt the salt which is then solidified to obtain the bamboo-salt. This bamboo-salt thus produced is mixed with the extract from the cortex of elm tree in the weight ratio of 5:7 and the mixture is formulated into an ointment which is effective for the prevention of dental caries.

However, although the bamboo-salt produced according to the method disclosed in Korean Early Published Patent Publication No. 90-4318 contains 34.6% of sodium, 55.6% of chlorine, 5.39% of water-insoluble material, 1.21% of magnesium, 0.17% of calcium, 0.98% of potassium, 6.20% of sulfate and 0.0001% of arsenic and has 6.43% of a drying loss, this is also lacking in essential mineral components and further does not contain the constituents in constant amounts.

Furthermore, since the bamboo-salt used in the toothpaste composition is a bamboo-salt prepared through three calcination procedures, its pharmacological efficacy is not sufficient. In addition, since the bamboo-salt is mixed with the elm tree extract in the weight ratio of 5:7 and then formulated into the ointment in the kneading state, the ointment is solidified within 2 hours after its preparation so that it cannot be well dispersed in the oral cavity, and therefore cannot properly display the role of tooth paste.

In addition, according to a method disclosed in Korean Patent Publication No. 94-64, a triennial large bamboo is cut down in the shape that the one end is opened and the other end is closed. The bay salt is hardened by pounding into the bamboo tube, and then the opened end of the bamboo tube is sealed with yellow earth which is kneaded with water. The bamboo tube is then calcined in the pottery furnace which is made of the yellow earth, at the internal temperature of 1000°C or more, in which pine tree and pine resin are used as a fuel. The heated product is allowed to cooled down naturally without using any cooling means and the salt masses thus obtained are collected and finely ground again. The ground salt is put again into the fresh bamboo tube in the same manner as above, and then the above calcining procedure with heat is repeatedly practiced eight times. In the ninth calcining procedure, in order to obtain a high temperature of 1500°C or more a special stainless furnace is used. The above furnace is heated by using pine resins only to melt and flow down the salt product. The melted salt product is solidified to obatin the desired bamboo-salt. The bamboo-salt thus obtained contains sodium chloride, iron, calcium, potassium, magnesium, manganese, phosphorus, zinc, sulfur, etc. The object of the above Korean Patent Publication is to provide a composition for enhancing oral hygiene, which contains the bamboo-salt obtained through nine calcinations according to the same manner as above, alone or in combination with sodium chloride in an amount of 1 to 15 wt%, and exhibits a good effect of preventing and treating periodontal diseases and preventing dental caries.

However, according to Korean Patent Publication No. 94-64, since in the process for preparing bamboo-salt from bamboo and salt the calcining procedure is carried out eight times at 1000°C or more, from the beginning, for 12 hours each time and the ninth calcining procedure is practiced using pine resin only as a fuel to make the salt flowing down, the bamboo-salt thus prepared is lacking in essential mineral components and does not contain each constituent in a constant amount. In addition, the toothpaste composition containing such bamboo-salt has also some disadvantages in that the pharmacological effect and the feeling of use are not sufficient.

Meanwhile, Korean Patent Publication No. 92-11470 also discloses a method for preparing bamboo-salt. According to this method, a large bamboo (triennial bamboo) grown in the sea wind is cut off in the shape that the one end is opened and the other end is closed. The bay salt is hardened by pounding into the bamboo tube, and then the pine needles, mugworts (collected in Gangwha province) and bamboo leaves are charged thereto in depth of about 1 cm. Then, the yellow earth free of manure collected in the mountain is passed nine times through a fine sieve, dried in the shade, and thereafter thickly kneaded. The bamboo tube is sealed up with the above kneaded yellow earth in depth of about 2 cm from the opened end of the bamboo tube. A large number of such bamboo tube are prepared in the same nanner as above and then heated in the pottery kiln which is made of the yellow earth, at the interanl temperature of 1000°C of more, in which pine trees and pine resins are used as a fuel. After 24 hours, the heated products are taken out of the kiln. At this time, the salt posts absorbed components of bamboos, pine needles, mugworts and yellow earth remain only. Then, the salt posts thus obtained are finely ground, and the ground salts are put into the bamboo tubes in the same manner above, and then the above heating process is repeated eight times. In the ninth heating process, in order to obtain a high temperature (1500°C or more), a special stainless furnace installed under ground is used. The above furnace is heated by using pine resins only to melt and flow down the salt products. The melted salt products are cooled down to obtain the desired bamboo-salt. The bamboo-salt thus obtained from the above procedure contains 90 to 98% of sodium chloride, 0.04 to 0.20% of iron, 0.1 to 0.9% of calcium, 0.01 to 1.5% of potassium, 0.40 to 1.10% of magnesium, about 0.006% of strontium, about 0.005% of manganese, about 0.002% of zinc, about 0.002% of fluorine, 0.015 to 0.400% of phosphate, 0.160 to 1.500% of sulfate, etc.

However, the bamboo-salts prepared according to said various methods have commonly some disadvantages in that the composition of the bamboo-salts is not constant in its constituents and further the pharmacological effect of the bamboo-salt is also not uniform.

Thus, the present inventors have extensively and widely studied to improve the problems involved in the prior known methods as mentioned above and found that a certain unique method as defined below, which differs from the prior methods, can produce a bamboo-salt which provides a uniform composition in its constituents and exhibits an excellent pharmacological effect. In addition, it could be identified that a composition for enhancing oral hygiene which contains such a bamboo-salt prepared by such a unique method, shows a superior effect for improving oral hygiene. According to this, we have completed the present invention.

Accordingly, it is an object of the present invention to provide a unique process for preparing bamboo-salt, as defined below.

It is another object of the present invention to provide a unique process for preparing bamboo-salt, characterized in that the bay salt is packed into a tube of non-dried large bamboo grown for 3 to 5 years in the weight ratio of about 1:2 to 1:5 (bamboo:salt), the opened end of the bamboo tube is sealed with yellow earth, the sealed bamboo tubes are loaded in 3 to 15 layers in the furnace and then subjected to the two-step calcination procedure, wherein the first step is carried out by heating at about 150 to 550°C for about 1 to 5 hours and the second step is carried out by heating at about 600 to 800°C for about 2 to 10 hours, to obtain the calcined salt, which is ground, packed into the fresh bamboo tube and then repeatedly calcined more seven times in the same manner as above, and finally the salt which is calcined eight times is melted by heating at about 1000 to 1800°C in the ninth calcining procedure.

In addition, it is still another object of the present invention to provide a bamboo-salt which contains about 85 to 98 wt% of sodium chloride (NaCl), about 1.5 to 10.0 wt% of potassium (K), about 0.2 to 1.0 wt% of phosphate ($PO_4$) and about 0.005 to 0.3 wt% of sulfate ($SO_4$) as the main component and further contains at least five (5) inorganic ionic components selected from magnesium, chrome, iron, silicon, aluminum, zinc, fluorine, copper, barium, manganese and nickel, and also magnesium oxide and/or magnesium silicate in the water-insoluble part.

Further, it is another object of the present invention to provide a composition for enhancing oral hygiene, which contains the said bamboo-salt in an amount of about 0.5 to 15 wt% with respect to the total weight of the composition.

The foregoing has outlined some of the more pertinent objects of the present invention. These objects should be construed to be merely illustrative of some of the more pertinent features and applications of the invention. Other many beneficial results can be obtained by applying the disclosed invention in a different manner or modifying the invention within the scope of the disclosure. Accordingly, other objects and a more thorough understanding of the invention may be had by referring to the disclosure of invention, in addition to the scope of the invention defined in the claims.

DISCLOSURE OF INVENTION

In one aspect, the present invention relates to a process for preparing bamboo-salt. More specifically, the present invention relates to a process for preparing bamboo-salt, characterized in that the bay salt is packed into a tube of non-dried large bamboo grown for 3 to 5 years in the weight ratio of about 1:2 to 1:5 (bamboo:salt), the opened end of the bamboo tube is sealed with yellow earth, the sealed bamboo tubes are loaded in 3 to 15 layers in the furnace and then subjected to the two-step calcination procedure, wherein the first step is carried out by heating at about 150 to 550°C for about 1 to 5 hours and the second step is carried out by heating at about 600 to 800°C for about 2 to 10 hours, to obtain the calcined salt, which is ground, packed into the fresh bamboo tube and then repeatedly calcined more seven times in the same manner as above, and finally the salt which is calcined eight times is melted by heating at about 1000 to 1800°C in the ninth calcining procedure.

In the second aspect, the present invention relates to a bamboo-salt which contains about 85 to 98 wt% of sodium chloride (NaCl), about 1.5 to 10.0 wt% of potassium (K), about 0.2 to 1.0 wt% of phosphate ($PO_4$) and about 0.005 to 0.3 wt% of sulfate ($SO_4$) as the main component and further contains at least five (5) inorganic ionic components selected from magnesium, chrome, iron, silicon, aluminum, zinc, fluorine, copper, barium, manganese and nickel, and also magnesium oxide and/or magnesium silicate in the water-insoluble part.

Further, in the third aspect, the present invention relates to a composition for enhancing oral hygiene, which contains the said bamboo-salt in an amount of about 0.5 to 15 wt% with respect to the total weight of the composition.

Hereinafter, the process for preparing bamboo-salt according to the present invention will be more specifically explained.

First, the bay salt is tightly packed into the bamboo tube, of which the one end is opened and the other end is closed, in the weight ratio of about 1:2 to 1:5 (bamboo:salt). Then, the bamboo tube is sealed with the kneaded yellow earth at the opened end and vertically loaded in 3 to 15 layers in the furnace. The furnace is heated by making a fire with pine tree at the fuel intake positioned on the lower part of the furnace. At this time, the furnace is heated for about 2 to 5 hours in the manner such that the internal temperature of the furnace is maintained in the range of about 150 to 550°C in the first step. Preferably, the first step is carried out by feeding a fire with pine tree for about 2 to 3 hours while maintaining the internal temperature of about 200 to 450°C.

In the second step, the bamboo tube containing the salt is calcined at about 600 to 800°C for about 2 to 10 hours. Preferably, the second step is conducted by heating at about 700 to 800°C for about 5 to 7 hours. At this time, it should be noted that the smoke rising from the burning of pine tree can be entered into the furnace but the flame of the burning pine tree should be kept out from entrance into the furnace so that the bamboo tube cannot be directly burned with flame. When the two calcination steps are completed, the calcined salt is cooled down by standing it in the natural state.

Then, the calcined salt post is separated by completely removing ash and yellow earth which was used for sealing the opened end of bamboo tube, and roughly ground in a suitable size. This ground salt is packed into the fresh bamboo tube in the same ratio as in the first procedure, and the opened end of the bamboo tub is sealed with yellow earth and then the bamboo tube is repeatedly calcined seven times in the same manner as the first procedure.

Finally, the ninth calcining procedure is carried out by filling the bamboo tube with the salt which is eight times calcined in the same manner as above, closing the opened end of the bamboo tube with yellow earth and then heating the bamboo tube at about 1000 to 1800°C in the furnace using pine tree and pine resin as a fuel to cause the calcined salt to melt and flow down.

4

The process for preparation of bamboo-salt according to the present invention can be conducted in such a manner that in each of the first to eighth calcination procedures the first step is practiced by heating the salt at about 200 to 450°C using a fire with pine tree for about 2 to 3 hours and the second step is carried out by heating the salt at about 700 to 800°C for about 5 to 7 hours, and then the ninth calcination procedure is practiced by heating the salt at about 1300 to 1600°C with a fire using pine tree and pine resin as a fuel.

In addition, the process for preparation of bamboo-salt according to the present invention can also be practiced in such a manner that in the first to eighth calcining procedures the bamboo tube containing the salt should not be directly burned by a fire with pine tree but can be calcined by a self-flame of bamboo which is ignited with heat and flame of a fire with pine tree, and the heat and flame of a fire with pine tree can be directly entered into the furnace.

When the first to eighth calcining procedures are conducted directly at about 800°C or more using a fire with pine tree, not through two-step procedure, and the bamboo is completely burned by a fire with pine tree, the content of potassium and phosphate in the bamboo-salt thus prepared is lower than that in the bamboo-salt prepared according to the present invention. In addition, when the ninth calcining procedure is conducted at the temperature lower than 1000°C, the bamboo-salt thus prepared contains the sulfate in an excessive amount (0.3 wt% or more).

The fuel which can used in the process for preparation of the bamboo-salt according to the present invention can be selected from pine tree, pine resin, electricity and gas.

The furnace which can be used in the process for preparation of the bamboo-salt according to the present invention can be made of yellow earth, firebrick, stainless, etc.

The bamboo-salt prepared according to the process of the present invention contains about 85 to 98 wt% of sodium chloride (NaCl), about 1.5 to 10.0 wt% of potassium, about 0.2 to 1.0 wt% of phosphate and about 0.3 to 0.005 wt% of sulfate as the main component, and further contains at least five (5) inorganic ions selected from magnesium, chrome, iron, silicon, aluminum, zinc, cupper, barium, nickel, manganese and fluorine, and at least one component selected from magnesium oxide and magnesium silicate in the water-insouble part.

The bamboo-salt prepared according to the process of the present invention contains each constituent in a uniform content, and shows excellent bactericidal and anti-inflammatory effects due to a synergistic action of sodium chloride and various inorganic ions and, therefore, can be effectively used for prevention and treatment of oral diseases, alleviation of various inflammatory symptoms, treatment of skin diseases, and the like.

A composition for enhancing oral hygiene according to the present invention is characterized in that it contains the bamboo-salt prepared according to the process of the present invention in an amount of about 0.5 to 15 wt% with respect to the total weight of the composition.

In the composition for enhancing oral hygiene according to the present invention, the bamboo-salt is preferably present in an amount of about 0.5 to 15 wt% with respect to the total weight of the composition. When the content of the bamboo-salt is lower than 0.5 wt%, the effect of the composition of preventing and treating periodontal diseases and of preventing dental caries is not sufficient, whereas when the content of the bamboo-salt exceeds 15 wt%, the use of the composition may be deniable due to a unique sulfide odor of the bamboo-salt and the unpleasant appearance and feeling of use of the composition.

The content of the bamboo-salt in the composition for enhancing oral hygiene according to the present invention is more preferably about 1.0 to 5.0 wt% with respect to the total weight of the composition. Particularly, to provide the composition for enhancing oral hygiene which is most effective for prevention and treatment of periodontal diseases and for prevention of dental caries and has the best feeling of use, the content of the bamboo-salt is most preferably about 2.0 to 5.0 wt% with respect to the total weight of the composition.

The composition for enhancing oral hygiene according to the present invention can further contain polishing agents, humectants, therapeutic agents, binders, foaming agents, preservatives, sweetening agents, flavouring agents, pH regulators, whitening agents, etc. depending on its formulation type and purpose of use.

As the polishing agent, dicalcium phosphate, hydrated silica, calcium carbonate, alumina hydroxide, kaolin, sodium bicarbonate(NaHCO$_3$), and the like can be used, with dicalcium phosphate, hydrated silica, calcium carbonate or sodium bicarbonate being preferable. The polishing agent may be any one selected from the above mentioned polishing agents or a mixture of two or more selected therefrom, and can be used in an amount of about 20 to 60 wt%, preferably in an amount of about 20 to 50 wt%, with respect to the total weight of the composition.

The humectant can be any one selected from glycerin, sorbitol, non-crystalline sorbitol solution, propylene glycol, polyethylene glycol and xylitol or a mixture of two or more selected therefrom and is preferably present in an amount of about 20 to 60 wt% with respect to the total weight of the composition. Preferably, as the humectant a mixture of glycerin or non-crystalline sorbitol solution and propylene glycol or polyethylene glycol in the weight ratio of about 10:1 to 1:1 can be used in an amount of about 20 to 50 wt% with respect to the total weight of the composition.

As the binder, one selected from carrageenan, xanthan gum, sodium carboxymethyl cellulose, carboxyvinyl polymer, sodium alginate, beegum and laponite or a mixture of two or more selected therefrom can be suitably used, with carrageenan or sodium carboxymethyl cellulose alone or a mixture of carrageenan and sodium carboxymethyl cellulose being more preferably used. The binder is used in an amount of about 0.1 to 3.0 wt%, preferably in an amount of about 0.5 to 2.0 wt%, with respect to the total weight of the composition.

As the foaming agent, one selected from an anionic surfactant such as sodium lauryl sulfate or sodium lauryl sarcosinate, sorbitan fatty acid ester, polyoxyethylene hardened castor oil, polyoxyethylene-polyoxypropylene based condensation polymer, and the like, or a mixture of two or more selected therefrom can be preferably used, with sodium lauryl sulfate or polyoxyethylene-polyoxypropylene condensation polymer alone or a mixture of sodium lauryl sulfate and polyoxyethylene-polyoxypropylene condensation polymer being more preferably used. When the mixture of sodium lauryl sulfate and polyoxyethylene-polyoxypropylene condensation polymer is used as the foaming agent, sodium lauryl sulfate and polyoxyethylene-polyoxypropylene condensation polymer can be used in a mixing ratio of about 5:1 to 1:5 on the basis of their weight. The foaming agent can be used in an amount of about 0.5 to 5.0 wt%, preferably in an amount of about 0.5 to 3.5 wt%, with respect to the total weight of the composition.

The sweetening agent can be one selected from sodium saccharin, aspartame and glycyrrhizic acid or a mixture of two or more selected therefrom and preferably used in an amount of about 0.05 to 0.5 wt% with respect to the total weight of the composition.

As the preservative, one selected from paraoxybenzoic acid esters and sodium benzoate or a mixture of two or more selected therefrom can preferably be used. As the therapeutic agent, vitamins, sodium fluoride, sodium fluorophosphate, chlorhexidine, triclosan, tranexamic acid, allantoins, caproic acids, and the like can be prefrably used.

As the flavouring agent, peppermint oil, spearmint oil, menthol, carvone, and the like can be preferably used in a suitable mixing ratio. More preferably, the mixture of said flavouring agents in a certain mixing ratio can be further mixed with anise oil in a suitable amount.

As the pH regulator, phosphoric acid, sodium phosphate, citric acid, sodium citrate, succinic acid, sodium succinate, tartaric acid, sodium tartarate, and the like, preferably phosphoric acid, sodium phosphate, citric acid, sodium citrate, etc., can be used to adjust the pH value to about 5 to 8, preferably about 6 to 8.

As the whitening agent titanium oxide can be preferably used in an amount of about 0.1 to 2 wt%, preferably in an amount of about 0.1 to 1.0 wt%, with respect to the total weight of the composition.

The composition for enhancing oral hygiene according to the present invention can be formulated in the form of a toothpaste, an oral detergent, a chewing gum, a teethridge massage cream, and the like. The composition according to the present invention can be used in a conventional amount, per day, which can be used in 1 to 6 times.

The present invention will be more specifically explained on the basis of the following examples and comparative examples. However, it should be understood that the technical scope of the present invention is not limited by these examples in any manner.

EXAMPLES 1 and 2

Examples 1 and 2 are the bamboo-salts prepared according to the process of the present invention, Comparative Examples 1 and 2 are two kinds of typical commercial bamboo-salts prepared according to the konwn method, and Comparative Example 3 is the common salt obtained by purifying the bay salt as the raw material for preparing the bamboo-salt. The bamboo-salts of the examples and comparative examples were prepared in the following manner and then used in the experiments for comparison of constituents and bactericidal activity.

(1) Preparation of the bamboo-salts of Examples 1 and 2:
The bay salt was tightly packed into a tube of triennial bamboo in the weight ratio of 1:2 to 1:5 (bamboo:salt) and then the opened end of the bamboo tube was sealed with the kneaded yellow earth. The bamboo tube was introduced into the furnace using pine tree as the fuel and heated at the internal temperature of the furnace of 150 to 550°C for 2 to 5 hours in the first step and then calcined at 600 to 800°C for 2 to 10 hours in the second step. Then, the calcined bamboo tube was allowed to stand in the natural state to cool down. The calcined salt post thus prepared was ground, packed into the fresh bamboo tube in the same manner as above and repeatedly calcined more seven (7) times to calcination procedure. Finally, in the ninth calcination procedure the salt which is calcined eight (8) times was packed into the bamboo tube and heated to 1000 to 1800°C in the furnace using pine tree and pine resin as a fuel to allow the salt to melt and flow down. The melted salt was cooled down to obtain the desire bamboo-salts.

(2) Preparation fo the bamboo salt of Comparative Example 1: (based on Korean Early Published Patent Publication No. 90-4318)
The bay salt was packed into the bamboo tube and the opend end of the bamboo tube was sealed with yellow earth. The bamboo tube was calcined two times with charcoal fire. The calcined salt thus obtained was ground to powder which was then packed again into the bamboo tube. The opened end of the bamboo tube was sealed with yellow earth. The bamboo tube was introduced into the furnace using pine resin as a fuel and heated at 1000°C to melt the salt. The melted salt was cooled down to obtain the bamboo salt.

(3) Preparation of the bamboo-salt of Comparative Example 2: (based on Korean Patent Publication No. 94-64)

The bay salt was hardened by pounding into the tube of triennial large bamboo and the opened end of the bamboo tube was sealed with the kneaded yellow earth. The bamboo tube was heated in the furnace which is made of the yellow earth, at the internal temperature of 1000°C or more in which pine tree and pine resin are used as a fuel. The calcined salt product was cooled down in the natural state. The salt post thus obtained was ground, packed into the fresh bamboo tube in the same manner as above and then repeatedly calcined further seven (7) times. In the ninth procedure, the salt which is calcined eight (8) times was packed into the fresh bamboo tube in the same manner as above. The bamboo tube was heated at 1500°C or more in the furnace using pine resin only as a fuel to melt and flow down she salt. The melted salt was cooled down to obatin the desired bamboo salt.

(4) preparation of the common salt of Comparative Example 3:

The bay salt was treated with an ion exchange membrane to contain the common salt.

EXPERIMENT 1 - Comparison of the constituents

The bamboo-salts of Examples 1 and 2, the bamboo-salts of Comparative Examples 1 and 2, and the common salt of Comparative Example 3 as prepared above were analysed according to the known methods for determining inorganic metal ions, i.e. the method using ion chromatography (IC), inductively coupled plasma (ICP), electronic diffraction X-ray (EDX) or ion analyzer (IA). The results are described in the following Table 1.

EP 0 663 207 B1

Table 1

(unit : ppm)

| Component | Examples | | Comparative Examples | | |
|---|---|---|---|---|---|
| | 1 | 2 | 1 | 2 | 3 |
| Sodium chloride | 94.8% | 93.5% | 96.0% | 96.6% | 96.8% |
| Calcium (Ca) | 300 | 650 | 1600 | 1900 | 820 |
| Iron (Fe) | 430 | 670 | 130 | 980 | 10 |
| Magnesium (Mg) | 4400 (0.44%) | 5100 (0.51%) | 5600 (0.56%) | 11300 (1.13%) | 14300 (1.43%) |
| Potassium (K) | 24600 (2.46%) | 21800 (2.18%) | 11000 (1.10%) | 14010 (1.40%) | 10800 (1.08%) |
| Chrome (Cr) | 50 | 70 | $\leq 10$ | $\leq 10$ | $\leq 10$ |
| Silicon (Si) | 200 | 180 | 250 | 3000 | 180 |
| Aluminum (Al) | 150 | 140 | 570 | 300 | No |
| Zinc (Zn) | 30 | 20 | $\leq 10$ | 10 | $\leq 10$ |
| Cupper (Cu) | 10 | 10 | $\leq 10$ | 10 | No |
| Barium (Ba) | 30 | 30 | $\leq 10$ | 20 | - |
| Nickel (Ni) | 50 | 60 | 10 | 30 | - |
| Manganese (Mn) | 60 | 50 | 10 | 40 | 10 |
| Boron (B) | 40 | 50 | No | No | No |
| Cobalt (Co) | 50 | 50 | 50 | 50 | 40 |
| Bromine (Br) | 40 | 40 | 500 | - | 600 |
| Fluorine (F) | 20 | 30 | No | 10 | $\leq 10$ |
| Sulfate ($SO_4$) | 1700 (0.17%) | 1700 (0.17%) | 8200 (0.82%) | 7200 (0.72%) | $\leq 10$ |
| Phosphate ($PO_4$) | 2600 (0.26%) | 2600 (0.26%) | $\leq 1000$ ($\leq 0.1$%) | $\leq 1000$ ($\leq 0.1$%) | $\leq 1000$ ($\leq 0.1$%) |
| Lead (Pb) | $\leq 0.1$ | $\leq 0.1$ | $\leq 0.1$ | $\leq 0.1$ | $\leq 0.1$ |
| Arsenic (As) | $\leq 0.1$ | $\leq 0.1$ | $\leq 0.1$ | $\leq 0.1$ | $\leq 0.1$ |

```
Note : (1) No = Not detected
       (2) -  = Not tested
```

EXPERIMENT 2 - Comparative test for bactericidal activity

The bamboo-salts of Examples 1 and 2, the bamboo-salts of Comparative Examples 1 and 2, and the common salt of Comparative Example 3 were tested for their bactericidal activity. According to a conventional method for determining minimum inhibitory concentration against anerobic and aerobic bacteria, causative organisms of oral diseases, i.e. Rothia dentocariosa (ATCC 19426), Actinomyces viscosus (KTCT 9146) and Streptococcus mutans (MDR 6715), and general strain, Escherichia coli (ATCC 8739) were respectively inoculated on BHI (Brain Heart Infusion) medium containing each of the test samples, in the concentration of $10^6$ CFU/ml. The medium was cultured for one day at 37°C. Then, the minimum inhibitory concetration (MIC) of the test samples at which the growth of test strains is inhibited was determined. The results are described in the following Table 2.

Table 2. Minimum Inhibitory Concentration against test strains

(unit : %)

| Test samples<br>Test Strains | Example | | Comparative Example | | |
|---|---|---|---|---|---|
| | 1 | 2 | 1 | 2 | 3 |
| Rothia dentocariosa | 2 | 3 | 5 | 3 | 5 |
| Actinomyces viscosus | 2 | 2 | 5 | 4 | 6 |
| Streptococcus mutans | 2 | 2 | 6 | 3 | 7 |
| Escherichia coli | 3 | 4 | 7 | 5 | 8 |

As can be seen from the result described in Table 2, the bamboo-salts prepared according to the process of the present invention (Examples 1 and 2) have an excellent inhibitory activity against the growth of microorganisms which is higher than that of the bamboo-salts prepared according to the known methods (Comparative Examples 1 and 2) and the common salt as the raw matrerial used in the general salt toothpaste (Comparative Example 3).

EXAMPLES 3 to 10 and COMPARATIVE EXAMPLES 4 to 7

The toothpaste compositions were prepared from the components described in the following Table 3 by dispersing powdery components including sodium carboxymethyl cellulose, saccharin and the preservative in non-crystalline sorbitol solution, diluting the mixture with purified water, further blending the mixture in the first mixer, adding the polishing agent such as dicalcium phosphate and the therapeutic agent to the mixture, finally adding the foaming agent such as sodium alkylsulfate, the stabilizer and the flavouring agent to the mixture, and then blending the resulting mixture under vacuum condition.

Table 3

(unit: %)

| COMPONENTS | EXAMPLE NO. | | | | | |
|---|---|---|---|---|---|---|
| | 3 | 4 | 5 | 6 | 7 | 8 |
| Dicalcium phosphate | 42.00 | 40.00 | 40.00 | - | - | 40.00 |
| Hydrated silica | - | - | - | 25.00 | - | - |
| Calcium carbonate | - | - | - | - | 38.00 | - |
| Sodium bicarbonate | - | - | - | - | 5.00 | - |
| Alumina hydroxide | 5.00 | 5.00 | - | 3.00 | - | - |
| Glycerin | - | - | 5.00 | - | 10.00 | - |
| Non-crystalline sorbitol solution(70%) | 35.00 | 28.00 | 25.00 | 25.00 | 25.00 | 10.00 |
| Propylene glycol | - | - | 5.00 | - | - | - |
| Polyethylene glycol | - | - | - | - | 3.00 | - |
| Xylitol | - | - | - | - | - | 10.00 |
| Sodium carboxymethyl cellulose | 1.00 | 1.00 | 1.00 | 1.00 | 1.00 | 1.00 |
| Sodium alkylsulfate | 1.50 | 1.50 | 1.50 | 1.50 | 1.50 | 1.50 |
| Polyoxyethylene-polyoxypropylene condensation polymer | 1.00 | 1.00 | 1.00 | 1.00 | 1.00 | 1.00 |
| Sodium saccharin | 0.10 | 0.10 | 0.10 | 0.10 | 0.10 | 0.10 |
| Paraoxybenzoic acid ester | 0.15 | 0.15 | 0.15 | 0.15 | 0.15 | 0.15 |
| Bamboo-salt A | 0.50 | 1.00 | 2.00 | 3.00 | 4.00 | 5.00 |
| Bamboo-salt C | - | - | - | - | - | - |
| Bamboo-salt D | - | - | - | - | - | - |
| Common salt | - | - | - | - | - | - |
| Titanium oxide | - | 0.50 | 0.50 | 0.50 | 0.50 | 0.50 |
| Sodium pyrophosphate | 0.20 | 0.20 | 0.20 | 0.20 | 0.20 | 0.20 |
| Flavouring agent | 1.00 | 1.00 | 1.00 | 1.00 | 1.00 | 1.00 |
| Purified water   add by | 100.00 | 100.00 | 100.00 | 100.00 | 100.00 | 100.00 |

## Table 3 (continued)

(unit : %)

| Component | EXAMPLE NO. | | COMPARATIVE EXAMPLE NO. | | | |
|---|---|---|---|---|---|---|
| | 9 | 10 | 4 | 5 | 6 | 7 |
| Dicalcium phosphate | 40.00 | 35.00 | 40.00 | 40.00 | 40.00 | 40.00 |
| Hydrated silica | - | - | - | - | - | - |
| Calcium carbonate | - | - | - | - | - | - |
| Sodium bicarbonate | - | - | - | - | - | - |
| Alumina hydroxide | - | - | - | - | - | - |
| Glycerin | - | - | - | - | - | - |
| Non-crystalline sorbitol solution(70%) | 35.00 | 20.00 | 30.00 | 30.00 | 30.00 | 30.00 |
| Propylene glycol | - | - | - | - | - | - |
| Polyethylene glycol | 5.00 | 5.00 | - | - | - | - |
| Xylitol | - | - | - | - | - | - |
| Sodium carboxymethyl cellulose | 1.00 | 1.00 | 1.00 | 1.00 | 1.00 | 1.00 |
| Sodium alkylsulfate | 1.50 | 1.50 | 1.50 | 1.50 | 1.50 | 1.50 |
| Polyoxyethylene-polyoxypropylene condensation polymer | 1.00 | 1.00 | - | - | - | - |
| Sodium saccharin | 0.10 | 0.10 | 0.10 | 0.10 | 0.10 | 0.10 |
| Paraoxybenzoic acid ester | 0.15 | 0.15 | 0.15 | 0.15 | 0.15 | 0.15 |
| Bamboo-salt A | 10.00 | 15.00 | - | - | - | - |
| Bamboo-salt C | - | - | 2.00 | - | - | - |
| Bamboo-salt D | - | - | - | 2.00 | - | - |
| Common salt | - | - | - | - | 2.00 | 15.00 |
| Titanium oxide | 0.50 | 0.50 | - | - | - | - |
| Sodium pyrophosphate | 0.20 | 0.20 | - | - | - | - |
| Flavouring agent | 1.00 | 1.00 | 1.00 | 1.00 | 1.00 | 1.00 |
| Purified water add by | 100.00 | 100.00 | 100.00 | 100.00 | 100.00 | 100.00 |

## EXPERIMENT 3

The toothpaste compositions of Examples 3 to 10 and Comparative Examples 4 to 7 were comparatively examined for their effects for inhibiting the formation of dental plaque and for alleviating teethridge inflammation according to the following procedure:

(1) The subjects for test were selected and allowed to hear an explanation of dental examination procedure.

(2) The subjects were randomly divided into twelve (12) groups so that each group can contain ten (10) subjects and allowed to receive education regarding the correct tooth brushing manner. Then, the dental states of the subjects were examined to determine a dental plaque index (Quigley-Hein Index modified by Turesky) and a gingivitis index (Gingival Bleeding Index).

(3) All the test groups were allowed to receive a teeth scaling.

(4) The test subjects were allowed to use the control toothpaste for two (2) weeks and then their dental state were examined to determine the dental plaque index and the gingivitis index which are used as the initial value.

(5) Each test group was allowed to use the respective toothpaste of Examples 3 to 10 and Comparative Examples 4 to 7, three times a day for four (4) weeks in the usual tooth brushing manner. After 2 weeks and 4 weeks, the dental states were examined to determine the dental plaque index and the gingivitis index.

(6) The test results were examined for the statistical significance by comparing the initial value of each test group with the value determined after 4 weeks according to a t-test.

The test results are described in the following Tables 4 and 5.

Table 4

| Dental Plaque Index | | | | |
|---|---|---|---|---|
| Test toothpaste | Initial value | After 2 weeks | After 4 weeks | Improved Index |
| Example 3 | 1.76±0.48 | 1.48±0.36 | 1.34±0.32 | 0.42±0.32 |
| Example 4 | 1.82±0.45 | 1.53±0.27 | 1.36±0.27 | 0.46±0.21 |
| Example 5 | 1.73±0.36 | 1.43±0.23 | 1.03±0.20 | 0.70±0.15 |
| Example 6 | 1.80±0.43 | 1.49±0.18 | 1.10±0.21 | 0.70±0.23 |
| Example 7 | 1.79±0.21 | 1.47±0.20 | 1.08±0.19 | 0.71±0.31 |
| Example 8 | 1.83±0.30 | 1.49±0.27 | 1.07±0.19 | 0.76±0.19 |
| Example 9 | 1.85±0.32 | 1.50±0.25 | 1.06±0.16 | 0.78±0.10 |
| Example 10 | 1.82±0.34 | 1.46±0.30 | 1.03±0.11 | 0.79±0.09 |
| Comparative Example 4 | 1.77±0.41 | 1.63±0.31 | 1.41±0.37 | 0.22±0.43 |
| Comparative Example 5 | 1.83±0.33 | 1.54±0.25 | 1.26±0.17 | 0.57±0.18 |
| Comparative Example 6 | 1.81±0.27 | 1.80±0.38 | 1.65±0.19 | 0.16±0.12 |
| Comparative Example 7 | 1.78±0.34 | 1.49±0.20 | 1.14±0.32 | 0.64±0.23 |
| Note :<br>Improved Index=(Initial value)-(value determined after 4 weeks) | | | | |

Table 5

| Gingivitis Index | | | | |
|---|---|---|---|---|
| Test toothpaste | Initial value | After 2 weeks | After 4 weeks | Improved Index |
| Example 3 | 1.32±0.32 | 1.10±0.23 | 0.92±0.14 | 0.40±0.16 |
| Example 4 | 1.32±0.21 | 1.09±0.19 | 0.83±0.23 | 0.49±0.24 |
| Example 5 | 1.29±0.10 | 0.95±0.21 | 0.67±0.24 | 0.62±0.19 |
| Example 6 | 1.30±0.17 | 0.93±0.30 | 0.68±0.20 | 0.62±0.23 |
| Example 7 | 1.34±0.20 | 0.95±0.27 | 0.70±0.16 | 0.64±0.14 |
| Example 8 | 1.32±0.25 | 0.93±0.24 | 0.68±0.27 | 0.64±0.26 |
| Example 9 | 1.30±0.25 | 0.90±0.21 | 0.65±0.20 | 0.65±0.12 |
| Example 10 | 1.31±0.21 | 0.90±0.16 | 0.64±0.30 | 0.67±0.24 |
| Comparative Example 4 | 1.31±0.17 | 1.21±0.31 | 1.05±0.29 | 0.26±0.31 |

Table 5 (continued)

| Gingivitis Index | | | | |
|---|---|---|---|---|
| Test toothpaste | Initial value | After 2 weeks | After 4 weeks | Improved Index |
| Comparative Example 5 | 1.30±0.16 | 1.00±0.26 | 0.80±0.36 | 0.50±0.32 |
| Comparative Example 6 | 1.29±0.14 | 1.26±0.37 | 1.24±0.32 | 0.05±0.41 |
| Comparative Example 7 | 1.31±0.24 | 0.95±0.23 | 0.74±0.33 | 0.57±0.39 |
| Note :<br>Improved Index=(Initial value)-(value determined after 4 weeks) | | | | |

From the results described in Tables 4 and 5, the followings can be confirmed:

a. According to the examination of statistical significance of the dental plaque index and the gingivitis index which are determined after four (4) weeks from the use of toothpastes of Examples 3 to 10 and Comparative Examples 4 to 7, it could be identified that the dental plaque index and the gingivitis index were significantly reduced after use of the toothpastes of Examples 3 to 10 and Comparative Examples 5 and 7. Accordingly, it could be determined that the toothpastes containing the bamboo-salts of Examples 3 to 10 according to the present invention or the known bamboo-salts and common salt of Comparative Examples 5 and 7 have a sigificant effect of alleviating dental plaque and gingivitis symptoms.

b. According to the examination of a significant difference between the toothpaste containing the bamboo-salts of the present invention (Example 5) and the toothpaste containing the known bamboo-salt (Comparative Example 5), it could be identified that they are significantly different from each other in their effects of alleviating dental plaque and gingivitis ($p < 0.05$). Accordingly, it could be confirmed that the toothpaste containing the bamboo-salt prepared according to the process of the present invention is superior to that containing the bamboo-salt prepared according to the known process in view of their pharmacological effects for dental plaque and gingivitis.

c. According to the examination of a significant difference between the toothpastes of Examples 4 and 5, it could be identified that they are significantly different from each other.

d. According to the examination of a significant difference between the toothpastes of Examples 5 to 10, the significant difference between the examined toothpastes was not identified ($p > 0.05$). However, it could be identified that the effect of the toothpaste increases in proportion to the concentration of the bamboo-salt contained therein.

EXPERIMENT 4

The feeling of use of the toothpastes of Examples 3 to 10 and Comparative Examples 4 to 7 were evaluated by making up a questionnaire to the user and comparing the obtained results.

(1) Test toothpaste groups
Toothpastes of Examples 3 to 10 and Comparative Examples 4 to 7

(2) Test subjects
20 Experts for questionnaire, aging from 20 to 40

(3) Test methods

(a) One toothpaste per each test toothpaste group was given to the experts and then they were allowed to use one toothpaste per hour in the usual tooth brushing manner.

(b) Evaluation was conducted according to a double blind method using a five (5) score comparing system. Five score comparing system:

```
5              4              3              2              1
|──────────|──────────|──────────|──────────|
very good    good       normal      bad      very bad
```

(c) Evalation item

- Degree of taste
- Degree of refreshment

(4) Test results (Table 6)

Table 6

| Test toothpaste | Degree of taste | Degree of refreshment |
|---|---|---|
| Example 3 | 3.25±0.58 | 2.25±0.42 |
| Example 4 | 3.27±0.49 | 3.50±0.39 |
| Example 5 | 4.01±0.34 | 4.20±0.26 |
| Example 6 | 3.43±0.41 | 4.36±0.33 |
| Example 7 | 3.05±0.39 | 4.32±0.23 |
| Example 8 | 3.00±0.35 | 4.13±0.45 |
| Example 9 | 2.65±0.29 | 3.05±0.32 |
| Example 10 | 2.31±0.66 | 2.45±0.52 |
| Comparative Example 4 | 3.42±0.34 | 3.56±0.27 |
| Comparative Example 5 | 3.44±0.28 | 3.58±0.36 |
| Comparative Example 6 | 3.17±0.41 | 3.54±0.38 |
| Comparative Example 7 | 1.98±0.42 | 2.01±0.42 |

(5) Conclusion

a. In the test regarding the degree of taste, it could be identified that the toothpastes of Examples 3 to 8 and Comparative Examples 4 to 6 have a normal to good taste and the toothpastes of Examples 9 and 10 and Comparative Example 7 have somewhat deniable taste. Accordingly, it could be determined that it is preferable to use the bamboo-salt and common salt in the toothpastes in an amount of below 5 wt% with respect to the total weight of the composition.

b. For the degree of refreshment, it could be identified that the toothpastes of Examples 4 to 8 and Comparative Examples 4 to 6 are good.

EXAMPLES 11 to 18 and COMPARATIVE EXAMPLES 8 to 11

1) Test compositions

Examples 11 to 18 and Comparative Examples 8 to 11 in the following Table 7 are the specific examples of the oral detergent composition. The respective oral detergent composition was prepared by completely dissolving a solubilizer in a solvent ethanol, adding a flavouring agent to the resulting solution, stirring thoroughly the mixture, adding the mixture to the purified water portion (a solution of a humectant and other components in purified water) which was previously prepared, with stirring to completely dissolve the constituents, and then filtering the resulting mixture according to a conventional method.

Table 7

(unit : %)

| Components | Example 11 | Example 12 | Example 13 | Example 14 |
|---|---|---|---|---|
| Ethanol | 10.00 | 10.00 | 10.00 | 10.00 |
| Polyoxyethylene-polyoxypropylene copolymer | 0.50 | 0.50 | 1.00 | - |
| Polyoxyethylene hardened castor oil | - | - | - | 1.00 |
| Concentrated glycerin | 10.00 | - | 15.00 | 15.00 |
| Sorbitol solution | - | 10.00 | - | - |
| Sodium saccharin | 0.01 | 0.01 | 0.01 | 0.01 |
| Disodium phosphate | 0.01 | 0.01 | - | - |
| Bamboo-salt A | 0.50 | 1.00 | 2.00 | 3.00 |
| Bamboo-salt C | - | - | - | - |
| Bamboo-salt D | - | - | - | - |
| Common salt | - | - | - | - |
| Cetylpyridinium chloride | 0.05 | 0.05 | 0.05 | 0.05 |
| Tranexamic acid | 0.05 | 0.05 | 0.05 | 0.05 |
| Tricronic acid | 0.10 | 0.10 | 0.10 | 0.10 |
| Flavouring agent | 0.10 | 0.10 | 0.10 | 0.10 |
| Purified water add by | 100.00 | 100.00 | 100.00 | 100.00 |

Table 7 (continued)

(unit : %)

| Components | Example 15 | Example 16 | Example 17 | Example 18 |
|---|---|---|---|---|
| Ethanol | 5.00 | 10.00 | 10.00 | - |
| Polyoxyethylene-polyoxypropylene copolymer | 0.50 | 1.00 | 1.00 | 2.00 |
| Polyoxyethylene hardened castor oil | 0.50 | - | - | - |
| Concentrated glycerin | 10.00 | 5.00 | 5.00 | 5.00 |
| Sorbitol solution | - | 5.00 | 5.00 | 10.00 |
| Sodium saccharin | 0.01 | 0.02 | 0.02 | 0.05 |
| Disodium phosphate | - | - | - | 0.1 |
| Bamboo-salt A | 4.00 | 5.00 | 10.00 | 15.00 |
| Bamboo-salt C | - | - | - | - |
| Bamboo-salt D | - | - | - | - |
| Common salt | - | - | - | - |
| Cetylpyridinium chloride | 0.05 | 0.05 | 0.05 | 0.05 |
| Tranexamic acid | 0.05 | 0.05 | 0.05 | 0.05 |
| Tricronic acid | 0.10 | 0.10 | 0.10 | 0.10 |
| Flavouring agent | 0.10 | 0.10 | 0.10 | 0.10 |
| Purified water add by | 100.00 | 100.00 | 100.00 | 100.00 |

Table 7 (continued)

(unit : %)

| Components | Comparative Example 8 | Comparative Example 9 | Comparative Example 10 | Comparative Example 11 |
|---|---|---|---|---|
| Ethanol | 10.00 | 10.00 | 10.00 | 10.00 |
| Polyoxyethylene-polyoxypropylene copolymer | 1.00 | 0.50 | 1.00 | 0.50 |
| Polyoxyethylene hardened castor oil | - | - | - | - |
| Concentrated glycerin | 10.00 | 15.00 | 10.00 | 15.00 |
| Sorbitol solution | - | - | - | - |
| Sodium saccharin | 0.01 | 0.01 | 0.01 | 0.05 |
| Disodium phosphate | - | - | - | - |
| Bamboo-salt A | - | - | - | - |
| Bamboo-salt C | 2.00 | - | - | - |
| Bamboo-salt D | - | 2.00 | - | - |
| Common salt | - | - | 2.00 | 15.00 |
| Cetylpyridinium chloride | 0.05 | 0.05 | 0.05 | 0.05 |
| Tranexamic acid | 0.05 | 0.05 | 0.05 | 0.05 |
| Tricronic acid | 0.10 | 0.10 | 0.10 | 0.10 |
| Flavouring agent | 0.10 | 0.10 | 0.10 | 0.10 |
| Purified water add by | 100.00 | 100.00 | 100.00 | 100.00 |

2) Test methods

The oral detergent compositions of Examples 11 to 18 and Comparative Examples 8 to 11 were comparatively examined for their effects of inhibiting the formation of dental plaque and of alleviating gingivitis symptoms. The results are described in the following Tables 8 and 9.

(1) Test procedures

(a) To comparatively examine the effect of the oral detergent compositions of inhibiting the formation of dental plaque and of alleviating gingivitis symptoms twenty (20) adult men and women were included in each group of the oral detergent compositions of Examples 11 to 18 and Comparative Examples 8 to 11.

(b) The subjects were allowed to brush the teeth with the usual toothpaste in the usual manner three times a day for four (4) weeks and to gargle with 10ml, per each time, of the oral test detergent compositions four (4) times per day, after tooth brushing and before retiring, for about 20 seconds each time. The dental plaque index (Quigley-Hein Index modified by Turesky) and the gingivitis index (Gingival Bleeding Index) were determined before and after the use of the oral detergent compositions.

(c) The test results were examined for their statistical significance according to a T-test by comparing the initial value with the value determined after 4 weeks from the use of the oral detergent compositions.

2) Test results

(1) Dental plaque index (Table 8)

Table 8

| Test toothpaste | Initial value | After 4 weeks | Improved Index |
|---|---|---|---|
| Example 11 | 1.82±0.45 | 1.43±0.35 | 0.39±0.22 |
| Example 12 | 1.90±0.50 | 1.45±0.42 | 0.45±0.24 |
| Example 13 | 1.87±0.34 | 1.25±0.26 | 0.62±0.15 |
| Example 14 | 1.79±0.47 | 1.10±0.20 | 0.69±0.20 |
| Example 15 | 1.85±0.20 | 1.13±0.23 | 0.72±0.09 |
| Example 16 | 1.93±0.46 | 1.19±0.18 | 0.74±0.12 |
| Example 17 | 1.87±0.24 | 1.10±0.19 | 0.77±0.08 |
| Example 18 | 1.88±0.21 | 1.10±0.34 | 0.78±0.30 |
| Comparative Example 8 | 1.91±0.42 | 1.72±0.45 | 0.19±0.23 |
| Comparative Example 9 | 1.77±0.38 | 1.28±0.30 | 0.49±0.18 |
| Comparative Example 10 | 1.87±0.41 | 1.65±0.40 | 0.22±0.23 |
| Comparative Example 11 | 1.79±0.35 | 1.21±0.54 | 0.58±0.40 |
| Note : Improved Index=(Initial value)-(value determined after 4 weeks) | | | |

(2) Gingivitis Index (Table 9)

Table 9

| Test toothpaste | Initial value | After 4 weeks | Improved Index |
|---|---|---|---|
| Example 11 | 1.35±0.25 | 0.98±0.22 | 0.37±0.15 |
| Example 12 | 1.32±0.32 | 0.84±0.25 | 0.48±0.17 |
| Example 13 | 1.33±0.27 | 0.70±0.17 | 0.63±0.16 |
| Example 14 | 1.29±0.31 | 0.65±0.30 | 0.64±0.09 |
| Example 15 | 1.34±0.22 | 0.65±0.19 | 0.69±0.12 |
| Example 16 | 1.30±0.22 | 0.56±0.15 | 0.74±0.10 |
| Example 17 | 1.33±0.23 | 0.55±0.17 | 0.78±0.12 |
| Example 18 | 1.32±0.33 | 0.52±0.30 | 0.80±0.31 |

Table 9 (continued)

| Test toothpaste | Initial value | After 4 weeks | Improved Index |
|---|---|---|---|
| Comparative Example 8 | 1.34±0.25 | 1.11±0.19 | 0.23±0.14 |
| Comparative Example 9 | 1.36±0.22 | 0.85±0.23 | 0.51±0.19 |
| Comparative Example 10 | 1.29±0.30 | 0.15±0.30 | 0.14±0.26 |
| Comparative Example 11 | 1.31±0.19 | 0.72±0.25 | 0.59±0.11 |
| Note :<br>Improved Index=(Initial value)-(value determined after 4 weeks) | | | |

3) Conclusion

a. According to the statistical examination of a significant difference between the dental plaque index and gingivitis index after use of the oral detergent compositions of Examples 11 to 18 and Comparative Examples 8 to 11 for four (4) weeks, it could be identified that the dental plaque index and the gingivitis index were siginificantly reduced after use of the oral detergent compositions of Examples 11 to 18 and Comparative Examples 9 and 11 for four (4) weeks.

b. As the result of the comparison of the effect of the oral detergent composition containing the bamboo-salt of the present invention (Example 13) of inhibiting the formation of dental plaque and of alleviating the gingivitis symptoms with that of the oral detergent composition containing the known bamboo-salt in the same amount (Comparative Example 9) on the basis of the dental plaque index and the gingivitis index, it could be identified that the effect of the oral detergent composition containing the bamboo-salt of the present invention is significantly higher than that of the oral detergent composition containing the known bamboo-salt ($p < 0.05$).

EXAMPLE 19 : Chewing gum

| Gum base | 25.0 wt% |
|---|---|
| Sorbitol | 44.0 wt% |
| Mannitol | 12.0 wt% |
| Glycerin | 13.0 wt% |
| Lexidine | 0.5 wt% |
| Sweetening agent | 2.0 wt% |
| Bamboo-salt | 2.0 wt% |
| Flavouring agent | 1.5 wt% |

EXAMPLE 20 : Teethridge massage cream

| Monolauryl glycerin | 3.0 wt% |
|---|---|
| Oleyl alcohol | 5.0 wt% |
| Polyethylene glycol | 15.0 wt% |
| White vaseline | 3.0 wt% |
| Sodium N-palmitic glutamate | 0.5 wt% |
| Hydroxyethyl cellulose | 5.0 wt% |

(continued)

| | |
|---|---|
| Tocopherol acetate | 0.1 wt% |
| Bamboo-salt | 3.0 wt% |
| Allantoin chlorohydroxy aluminum | 1.0 wt% |
| Sweetening agent | 0.2 wt% |
| Flavouring agent | 0.3 wt% |
| Purified water add by | 100.0 wt% |

Both compositions of Examples 19 and 20 show an excellent effect of preventing and treating periodontal diseases amd of preventing dental caries.

Although this invention has been described in its preferred form with a certain degree of particularity, it is appreciated by those skilled in the art that the present disclosure of the preferred form has been made only by way of example and that numerous changes in the details of the construction, combination and arrangement of parts may be resorted to without departing from the spirit and scope of the invention.

## Claims

1. A process for preparing bamboo-salt, characterized in that the bay salt is packed into a tube of non-dried large bamboo grown for 3 to 5 years in the weight ratio of about 1:2 to 1:5 (bamboo:salt), the opened end of the bamboo tube is sealed with yellow earth, the sealed bamboo tubes are loaded in 3 to 15 layers in the furnace and then subjected to the two-step calcination procedure, wherein the first step is carried out by heating at about 150 to 550°C for about 1 to 5 hours and the second step is carried out by heating at about 600 to 800°C for about 2 to 10 hours, to obtain the calcined salt, which is ground, packed into the fresh bamboo tube and then repeatedly calcined more seven times in the same manner as above, and finally the salt which is calcined eight times is melted by heating at about 1000 to 1800°C at the ninth calcining procedure.

2. The process according to claim 1, characterized in that the bamboo tube containing the salt is calcined in each of the first to eight calcination procedures by heating the salt at about 200 to 450°C using a fire with pine tree for about 2 to 3 hours in the first step and at about 700 to 800°C for about 5 to 7 hours in the second step and then in the ninth calcination procedure the salt which is calcined eight times is further calcined by heating the salt at about 1300 to 1600°C with a fire using pine tree and pine resin as a fuel.

3. The process according to claim 1, characterized in that in the first to eighth calcining procedures the bamboo tube containing the salt is not directly burned by a fire with pine tree but is calcined by a self-flame of bamboo which is ignited with heat and flame of a fire with pine tree and the heat and flame of a fire with pine tree is directly entered into the furnace.

4. The process according to claim 1 or 2, characterized in that the fuel for heating the bamboo tube containing the salt is selected from pine tree, pine resin, electricity and gas.

5. The process according to claim 1 or 2, characterized in that the furnace for preparing the bamboo-salt is made of yellow earth, firebrick or stainless.

6. A bamboo-salt, characterized in that it contains about 85 to 98 wt% of sodium chloride (NaCl), about 1.5 to 10.0 wt% of potassium (K), about 0.2 to 1.0 wt% of phosphate ($PO_4$) and about 0.005 to 0.3 wt% of sulfate ($SO_4$) as the main component and further contains at least five (5) inorganic ionic components selected from magnesium, chrome, iron, silicon, aluminum, zinc, fluorine, cupper, barium, manganese and nickel, and also magnesium oxide and/or magnesium silicate in the water-insoluble part.

7. A composition for enhancing oral hygiene, characterized in that it contains a bamboo-salt according to claim 6 in an amount of about 0.5 to 15 wt% with respect to the total weight of the composition.

8. The composition according to claim 7, characterized in that the bamboo-salt is present in an amount of about 1.0 wt% to 5.0 wt% with respect to the total weight of the composition.

9. The composition according to claim 8, characterized in that the bamboo-salt is present in an amount of about 2.0 wt% to 4.0 wt% with respect to the total weight of the composition.

10. The composition according to claim 7, characterized in that the composition for enhancing oral hygiene is in the form of a toothpaste.

11. The composition according to claim 7, characterized in that the composition for enhancing oral hygiene is in the form of an oral detergent.

12. The composition according to claim 7, characterized in that the composition for enhancing oral hygiene is in the form of a chewing gum.

13. The composition according to claim 7, characterized in that the composition for enhancing oral hygiene is in the form of a teethridge massage cream.

14. The composition according to claim 10, characterized in that it is the toothpaste composition which additionally contains one or more components selected from a polishing agent, a humectant, a therapeutic agent, a binder and a flavouring agent.

15. The composition according to claim 14, characterized in that it is the toothpaste composition in which the polishing agent is one or more components selected from dicalcium phosphate, hydrated silica, calcium carbonate, alumina hydroxide, kaolin and sodium bicarbonate and is present in an amount of about 20 to 50 wt% with respect to the total weight of the composition.

16. The composition according to claim 14, characterized in that it is the toothpaste composition in which the humectant is one or more components selected from glycerin, sorbitol, propylene glycol, polyethylene glycol and xylitol and is present in an amount of about 20 to 60 wt% with respect to the total weight of the composition.

17. The composition according to claim 16, characterized in that the humectant is a mixture of glycerin or non-crystalline sorbitol solution and propylene glycol in the weight ratio of about 10:1 to 1:1.

18. The composition according to any one of claims 10 to 13, characterized in that the therapeutic agent is one or more component selected from vitamins, sodium fluoride, sodium fluorophosphate, chlorhexidine, triclosan, tranexamic acid, allantoins and caproic acid and is present in an amount of about 0.01 to 1.0 wt% with respect to the total weight of the composition.

19. The composition according to any one of claims 10 to 13, characterized in that it additionally contains one or more flavouring agent selected from peppermint oil, spearmint oil, menthol, carvone and anise oil.

**Patentansprüche**

1. Verfahren zur Herstellung von Bambus-Salz, dadurch gekennzeichnet, daß Meersalz in ein Rohr von nicht-getrocknetem, großem Bambus mit einer Wachstumszeit von 3 bis 5 Jahren im Gewichtsverhältnis von etwa 1:2 bis etwa 1:5 (Bambus:Salz) eingefüllt wird, wobei das offene Ende des Bambus-Rohrs mit Gelberde verschlossen wird, die verschlossenen Bambus-Rohre in 3 bis 15 Schichten in den Ofen gebracht werden und sodann einem 2-stufigen Calcinierungsverfahren unterzogen werden, wobei die erste Stufe durch etwa 1- bis 5-stündiges Erwärmen auf etwa 150 bis 550°C und die zweite Stufe durch etwa 2- bis 10-stündiges Erwärmen auf etwa 600 bis 800°C durchgeführt wird, wodurch man ein calciniertes Salz erhält, das gemahlen, in ein frisches Bambus-Rohr gefüllt und sodann weitere siebenmal auf die vorstehend beschriebene Weise calciniert wird, wobei schließlich das Salz, das achtmal calciniert worden ist, beim neunten Calcinierungsvorgang durch Erwärmen auf etwa 1000 bis 1800°C geschmolzen wird.

2. Verfahren nach Anspruch 1, dadurch gekennzeichnet, daß das das Salz enthaltende Bambus-Rohr bei der ersten bis achten Calcinierung calciniert wird, indem man das Salz in der ersten Stufe etwa 2 bis 3 Stunden unter Verwendung eines Kiefernholzfeuers bei etwa 200 bis 450°C und in der zweiten Stufe etwa 5 bis 7 Stunden bei etwa 700 bis 800°C calciniert und anschließend bei der neunten Calcinierung das bereits achtmal calcinierte Salz einer weiteren Calcinierung unterwirft, indem man es mit einem Feuer unter Verwendung von Kiefernholz und Kiefernharz als Brennstoff auf etwa 1300 bis 1600°C erwärmt.

3. Verfahren nach Anspruch 1, dadurch gekennzeichnet, daß bei der ersten bis achten Calcinierung das Bambus-Rohr, das das Salz enthält, nicht direkt durch ein Kiefernholzfeuer verbrannt wird, sondern unter Selbstentzündung des Bambus calciniert wird, wobei der Bambus unter Wärme- und Flammeneinwirkung eines Kiefernholzfeuers entzündet wird und die Wärme und die Flamme eines Kiefernholzfeuers direkt in den Ofen gelangen.

4. Verfahren nach Anspruch 1 oder 2, dadurch gekennzeichnet, daß der Brennstoff zum Erwärmen des Bambus-Rohrs, das das Salz enthält, unter Kiefernholz, Kiefernharz, Elektrizität und Gas ausgewählt wird.

5. Verfahren nach Anspruch 1 oder 2, dadurch gekennzeichnet, daß der Ofen zur Herstellung des Bambus-Salzes aus Gelberde, feuerfesten Ziegelsteinen oder rostfreiem Stahl gefertigt ist.

6. Bambus-Salz, gekennzeichnet durch folgende Bestandteile: etwa 85 bis 98 Gew.-% Natriumchlorid (NaCl), etwa 1,5 bis 10,0 Gew.-% Kalium (K), etwa 0,2 bis 1,0 Gew.-% Phosphat ($PO_4$) und etwa 0,005 bis 0,3 Gew.-% Sulfat ($SO_4$) als Hauptbestandteile und zusätzlich mindestens 5 weitere anorganische ionische Komponenten, die unter Magnesium, Chrom, Eisen, Silicium, Aluminium, Zink, Fluor, Kupfer, Barium, Mangan und Nickel ausgewählt sind, sowie Magnesiumoxid und/oder Magnesiumsilicat im wasserunlöslichen Teil.

7. Zusammensetzung zur Unterstützung der Mundhygiene, dadurch gekennzeichnet, daß es ein Bambus-Salz nach Anspruch 6 in einer Menge von etwa 0,5 bis 15 Gew.-%, bezogen auf das Gesamtgewicht der Zusammensetzung, enthält.

8. Zusammensetzung nach Anspruch 7, dadurch gekennzeichnet, daß das Bambus-Salz in einer Menge von etwa 1,0 bis 5,0 Gew.-%, bezogen auf das Gesamtgewicht der Zusammensetzung, vorhanden ist.

9. Zusammensetzung nach Anspruch 8, dadurch gekennzeichnet, daß das Bambus-Salz in einer Menge von etwa 2,0 bis 4,0 Gew.-%, bezogen auf das Gesamtgewicht der Zusammensetzung, vorhanden ist.

10. Zusammensetzung nach Anspruch 7, dadurch gekennzeichnet, daß die Zusammensetzung zur Unterstützung der Mundhygiene in Form einer Zahnpasta vorliegt.

11. Zusammensetzung nach Anspruch 7, dadurch gekennzeichnet, daß die Zusammensetzung zur Unterstützung der Mundhygiene in Form eines oralen Detergens vorliegt.

12. Zusammensetzung nach Anspruch 7, dadurch gekennzeichnet, daß die Zusammensetzung zur Unterstützung der Mundhygiene in Form eines Kaugummis vorliegt.

13. Zusammensetzung nach Anspruch 7, dadurch gekennzeichnet, daß die Zusammensetzung zur Unterstützung der Mundhygiene in Form einer Zahnfleisch-Massagecreme vorliegt.

14. Zusammensetzung nach Anspruch 10, dadurch gekennzeichnet, daß es sich um eine Zahnpastazusammensetzung handelt, die zusätzlich einen oder mehrere Bestandteile enthält, die unter Poliermitteln, Feuchthaltemitteln, therapeutischen Wirkstoffen, Bindemitteln und Geschmacksstoffen ausgewählt sind.

15. Zusammensetzung nach Anspruch 14, dadurch gekennzeichnet, daß es sich um eine Zahnpastazusammensetzung handelt, bei der es sich beim Poliermittel um eine oder mehrere Komponenten, die unter Dicalciumphosphat, hydratisiertem Siliciumdioxid, Calciumcarbonat, Aluminiumoxid-hydroxid, Kaolin und Natriumbicarbonat ausgewählt sind, in einer Menge von etwa 20 bis 50 Gew.-%, bezogen auf das Gesamtgewicht der Zusammensetzung, handelt.

16. Zusammensetzung nach Anspruch 14, dadurch gekennzeichnet, daß es sich um eine Zahnpastazusammensetzung handelt, bei der es sich beim Feuchthaltemittel um eine oder mehrere Komponenten, die unter Glycerin, Sorbit, Propylenglykol, Polyethylenglykol und Xylit in einer Menge von etwa 20 bis 60 Gew.-%, bezogen auf das Gesamtgewicht der Zusammensetzung, ausgewählt sind, handelt.

17. Zusammensetzung nach Anspruch 16, dadurch gekennzeichnet, daß es sich beim Feuchthaltemittel um ein Gemisch aus Glycerin oder einer Lösung von nicht-kristallinem Sorbit und Propylenglykol im Gewichtsverhältnis von etwa 10:1 bis 1:1 handelt.

**18.** Zusammensetzung nach einem der Ansprüche 10 bis 13, dadurch gekennzeichnet, daß es sich beim therapeutischen Mittel um einen oder mehrere Bestandteile aus der Gruppe Vitamine, Natriumfluorid, Natriumfluorophosphat, Chlorhexidin, Triclosan, Tranexamsäure, Allantoine und Capronsäure in einer Menge von etwa 0,01 bis 1,0 Gew.-%, bezogen auf das Gesamtgewicht der Zusammensetzung, handelt.

**19.** Zusammensetzung nach einem der Ansprüche 10 bis 13, dadurch gekennzeichnet, daß sie zusätzlich einen oder mehrere Geschmackstoffe, die unter Pfefferminzöl, Krauseminzöl, Menthol, Carvon und Anisöl ausgewählt sind, handelt.

**Revendications**

**1.** Procédé pour préparer un sel-bambou, caractérisé en ce qu'on tasse du sel marin dans un tube de bambou de grande dimension, non séché, qui a poussé pendant 3 à 5 ans, selon un rapport pondéral d'environ 1:1 à 1:5 (bambou:sel), l'extrémité ouverte du tube de bambou est scellée avec de l'argile ocreuse, les tubes de bambou scellés sont placés en 3 à 15 couches dans le four, puis soumis à une opération de calcination en deux étapes, où la première étape est mise en oeuvre par chauffage à une température d'environ 150 à 550°C pendant environ 1 à 5 heures, et la deuxième étape est mise en oeuvre par chauffage à une température d'environ 600 à 800°C pendant environ 2 à 10 heures, pour donner un sel calciné, que l'on broie, que l'on tasse dans un tube de bambou frais puis que l'on calcine d'une manière répétitive encore 7 fois de la même manière que ci-dessus, puis le sel calciné 8 fois est fondu par chauffage à une température d'environ 1000°C à 1800°C au cours d'une neuvième opération de calcination.

**2.** Procédé selon la revendication 1, caractérisé en ce que le tube de bambou contenant le sel est calciné dans chacune des huit premières opérations de calcination, par chauffage du sel à une température d'environ 200 à 450°C, par utilisation d'un feu avec du bois de pin pendant environ 2 à 3 heures dans la première étape et à une température d'environ 700 à 800°C pendant environ 5 à 7 heures dans la deuxième étape, puis, au cours de la neuvième opération de calcination, le sel qui a été calciné huit fois est encore calciné par chauffage du sel à une température d'environ 1300 à 1600°C à l'aide d'un feu utilisant du bois de pin et de la résine de pin en tant que combustible.

**3.** Procédé selon la revendication 1, caractérisé en ce que, dans les huit premières opérations de calcination, le tube de bambou contenant le sel n'est pas directement brûlé par un feu utilisant du bois de pin, mais est calciné par la flamme spontanée du bambou, qui est enflammée sous l'effet de la chaleur et de la flamme d'un feu utilisant du bois de pin, la chaleur et la flamme d'un feu de bois de pin étant directement introduites dans le four.

**4.** Procédé selon la revendication 1 ou 2, caractérisé en ce que le combustible destiné à chauffer le tube de bambou contenant le sel est choisi parmi le bois de pin, la résine de pin, l'électricité et le gaz.

**5.** Procédé selon la revendication 1 ou 2, caractérisé en ce que le four destiné à préparer le sel-bambou est constitué d'argile ocreuse, de brique réfractaire ou d'un acier inoxydable.

**6.** Sel-bambou, caractérisé en ce qu'il contient d'environ 85 à 98 % en poids de chlorure de sodium (NaCl), d'environ 1,5 à 10,0 % en poids de potassium (K), d'environ 0,2 à 1,0 % en poids de phosphate ($PO_4$) et d'environ 0,005 à 0,3 % en poids de sulfate ($SO_4$) en tant que constituants principaux, et qui contient en outre au moins 5 composants ioniques minéraux choisis parmi le magnésium, le chrome, le fer, le silicium, l'aluminium, le zinc, le fluor, le cuivre, le baryum, le manganèse et le nickel, ainsi que de l'oxyde de magnésium et/ou du silicate de magnésium dans la partie insoluble dans l'eau.

**7.** Composition pour améliorer l'hygiène dentaire, caractérisée en ce qu'elle contient un sel-bambou selon la revendication 6 en une quantité d'environ 0,5 à 15 % en poids par rapport au poids total de la composition.

**8.** Composition selon la revendication 7, caractérisée en ce que le sel-bambou est présent en une quantité d'environ 1,0 % en poids à 5,0 % en poids par rapport au poids total de la composition.

**9.** Composition selon la revendication 8, caractérisée en ce que le sel-bambou est présent en une quantité d'environ 2,0 % en poids à 4,0 % en poids par rapport au poids total de la composition.

**10.** Composition selon la revendication 7, caractérisée en ce que la composition destinée à améliorer l'hygiène buccale se présente sous forme d'une pâte dentifrice.

**11.** Composition selon la revendication 7, caractérisée en ce que la composition destinée à améliorer l'hygiène buccale se présente sous forme d'un détergent pour utilisation buccale.

**12.** Composition selon la revendication 7, caractérisée en ce que la composition destinée à améliorer l'hygiène buccale se présente sous forme d'une gomme a mâcher.

**13.** Composition selon la revendication 7, caractérisée en ce que la composition destinée à améliorer l'hygiène buccale se présente sous forme d'une crème de massage de la crête des dents.

**14.** Composition selon la revendication 10, caractérisée en ce qu'il s'agit d'une composition de pâte dentifrice qui contient en outre un ou plusieurs composants choisis parmi un agent de polissage, un humectant, un agent thérapeutique, un liant et un aromatisant.

**15.** Composition selon la revendication 14, caractérisée en ce qu'il s'agit d'une composition de pâte dentifrice dans laquelle l'agent de polissage est constitué d'un ou plusieurs composants choisis parmi le phosphate dicalcique, la silice hydratée, le carbonate de calcium, l'hydroxyde d'alumine, le kaolin et le bicarbonate de sodium, et est présent en une quantité d'environ 20 à 50 % en poids par rapport au poids total de la composition.

**16.** Composition selon la revendication 14, caractérisée en ce qu'il s'agit d'une composition de pâte dentifrice dans laquelle l'humectant est constitué d'un ou plusieurs composants choisis parmi le glycérol, le sorbitol, le propylèneglycol, le polyéthylèneglycol et le xylitol, et est présent en une quantité d'environ 20 à 60 % en poids par rapport au poids total de la composition.

**17.** Composition selon la revendication 16, caractérisée en ce que l'humectant est un mélange de glycérol ou d'une solution de sorbitol non cristallin et de propylèneglycol selon un rapport pondéral d'environ 10:1 à 1:1.

**18.** Composition selon l'une quelconque des revendications 10 à 13, caractérisée en ce que l'agent thérapeutique est constitué d'un ou plusieurs composants choisis parmi les vitamines, le fluorure de sodium, le fluorophosphate de sodium, la chlorhexidine, le triclosan, l'acide tranexamique, les allantoïnes et l'acide caproïque, et est présent en une quantité d'environ 0,01 à 1,0 % en poids par rapport au poids total de la composition.

**19.** Composition selon l'une quelconque des revendications 10 à 13, caractérisée en ce qu'elle contient en outre un ou plusieurs aromatisants choisis parmi l'essence de menthe poivrée, l'essence de menthe verte, le menthol, la carvone et l'essence d'anis.